# EUROPÄISCHE PATENTSCHRIFT

(11) **EP 0 537 443 B1**
(45) Veröffentlichungstag und Bekanntmachung des Hinweises auf die Patenterteilung: **18.09.1996**
(21) Anmeldenummer: 92114012.5
(22) Anmeldetag: 17.08.1992
(51) Int. Cl.: C12P 13/08, C12N 15/01

(54) **Verfahren zur Erhöhung der Leistungsfähigkeit Aminosäuren ausscheidender Bakterien**
Process for the enhancement of the productivity of bacteria excreting amino acids
Procédé pour l'augmentation de la productivité des bactéries sécrétant des acides aminés

(30) Priorität: 18.10.1991 DE 4134450
(43) Veröffentlichungstag der Anmeldung: 21.04.1993
(73) Patentinhaber: Degussa Aktiengesellschaft, D-60311 Frankfurt (DE)
(72) Erfinder: Kircher, Manfred, Dr., W-4800 Bielefeld 1 (DE); Günther, Kurt, Dr., W-6455 Erlensee (DE); Bachmann, Bernd, Dr., W-4806 Werther (DE)

(56) Entgegenhaltungen:
- FR-A- 2 497 231
- PATENT ABSTRACTS OF JAPAN, unexamined applications, Sektion C, Band 7, Nr. 281, 15. Dezember 1983, The Patent Office Japanese Govt.; Seite 33 C 200/
- PATENT ABSTRACTS OF JAPAN, unexamined applications, Sektion C, Band 13, Nr. 504, 13. November 1989, The Patent Office Japanese Govt.; Seite 113 C 653/
- PATENT ABSTRACTS OF JAPAN, unexamined applications, Sektion C, Band 6, Nr. 98, 08. Juni 1982, The Patent Office Japanese Govt.; Seite 84 C 106/
- PATENT ABSTRACTS OF JAPAN, unexamined applications, Sektion C, Band 6, Nr. 98, 08. Juni 1982, The Patent Office Japanese Govt.; Seite 85 C 106/
- PATENT ABSTRACTS OF JAPAN, unexamined applications, Sektion C, Band 4, Nr. 186, 20. Dezember 1980, The Patent Office Japanese Govt.; Seite 4 C 36/
- PATENT ABSTRACTS OF JAPAN, unexamined applications, Sektion C, Band 9, Nr. 192. 8. August 1985, THE PATENT OFFICE JAPANESE GOVERNMENT Seite 135 C 296/

## Beschreibung

Die Erfindung betrifft ein Verfahren zur Erhöhung der Leistungsfähigkeit Aminosäuren, insbesondere L-Lysinausscheidender coryneformer Backterien.

Die essentielle Aminosäure L-Lysin ist als Nahrungsund Tierfutterzusatz, sowie als Wirkstoff und Bestandteil von pharmazeutischen Produkten von großer industrieller Bedeutung.

Für die Herstellung von L-Lysin ist die Fermentation das bedeutendste Verfahren. Vor allem coryneforme Bakterien der Gattungen Corynebacterium und Brevibacterieum werden in der Produktion eingesetzt. Durch Mutationen ist die Regulation der Lysin-Biosynthese dieser Stämme so verändert, daß sie Lysin über den Eigenbedarf hinaus produzieren und in das Medium ausscheiden.

Derartige Überproduzenten erhält man durch Suche nach Mutanten, in denen einzelne Schritte des Aminosäurestoffwechsels blockiert sind (z. B. Hse- oder Thr-Auxotrophe), die gegen ein Analogon von Lysin resistent sind oder weitere Mutationen enthalten. Hochleistungsstämme besitzen im allgemeinen eine oder mehrere Auxotrophien, eine oder mehrere Analoga-Resistenzen oder eine Kombination dieser Mutationen. Eine zusammenfassende Darstellung dieser Entwicklung von Lysin-Produzenten gebe 0. Tosaka und K. Takinami, (Progr. Ind. Microbiol. (Biotechnol. Amino Acids 24 (1986) 152 bis 172.

Die Leistungsfähigkeit eines Stammes wird nun dadurch bestimmt, daß der Kohlenstoff-Fluß des gesamten Stoffwechsels möglichst effizient in Richtung von Lysin gedrängt wird. Dies bedeutet inerseits die Aktivierung der Lysin-Biosynthese sowie die Beseitigung von Engpässen in diesem Bereich und andererseits die Blockierung von Stoffwechselwegen die zur Nebenproduktbildung führen. So beschreibt K. Nakayama für Lysinproduzenten die Bildung von Homoserin, Valin, Glutaminsäure, Lactat und Succinat ( in: The Microbial Production of Amino Acids; eds. K. Yamada, S. Kinoshita, T. Tsunoda, K. Aida; John Wiley & Sons (1972), 369 bis 398.

Aufgabe der Erfindung ist es, durch weitere Mutationen die Leistungsfähigkeit Aminosäuren ausscheidender, insbesondere Lysin ausscheidender coryneformer Bakterien zu steigern.

Gegenstand der Erfindung ist ein Verfahren zur Erhöhung der Leistungsfähigkeit von Aminosäuren, insbesondere L-Lysin ausscheidenden Stämmen coryneformer Mikroorganismen, das dadurch gekennzeichnet ist, daß man bei diesen Stämmen die Fähigkeit zum Wachstum auf Trehalose induziert. Bei Trehalose handelt es sich um alpha-D-Glucopyranosyl-alpha-D-Glucopyranosid. Am Beispiel von Brevibacterium flavum wurde gezeigt, daß Trehalose sowohl vom Wildtyp (R. Londoni, Th. Walker ; Bioscience Reports 5, 509 bis 515 (1985) als auch von einer Lysin ausscheidenden Mutante (L. Inbar, A. Lapidot; Eur. J. Biochem. 162, 621 bis 633 (1987) synthetisiert wird. Auch für Aminosäuren produzierende Stämme von Corynebacterium glutamicum wurde inzwischen die Bildung von Trehalose beschrieben (C.A. 116 (15) 150157j, JP 04004888-A2, nachveröffentlicht). In diesem Fall wird die Trehalose-haltige Glutaminsäure-Fermentationsbrühe mit dem Enzym Trehalose nachbehandelt.

Gleichzeitig kann man jedoch feststellen, daß coryneforme Bakterien wie Corynebacterium glutamicum (ATCC13032) auf Trehalose als C-Quelle nicht und Brevibacterium flavum (ATCC14067) und Brevibacterium lactofermentum ATCC13869 schwach wachsen.

Im vollautomatischen Identifizierugsgerät VITEK (Modell AMS/IMS), und auf OF-Testnährboden (Merck Art. 10282) ist die Säurebildung aus Trehalose für die drei Species nicht nachweisbar. In Phenolrot-Bouillon (Merck Art. 10987) zeigt sich für Brevibacterium flavum und lactofermentum eine schwach positive Reaktion, für Corynebacterium glutamicum ist keine Säurebildung aus Trehalose nachweisbar.

Obwohl bisher ein Zusammenhang zwischen der Eigenschaft coryneformer Bakterien, Trehalose als C-Quelle zu verwerten, und ihrer Fähigkeit, Aminosäuren auszuscheiden, nicht bekannt war, zeigt sich jetzt, daß coryneforme, Aminosäuren ausscheidende Bakterien, bei denen die Fähigkeit zum Wachstum auf Trehalose induziert wurde, eine gegenüber den Ausgangsstämmen gesteigerte Aminosäureproduktion aufweisen. Dies gilt insbesondere für Stämme, die ursprünglich auch Trehalose ausscheiden.

Dabei findet man gleichzeitig, daß die Trehalose verwertenden Mutanten bei der Kultivierung auf einer anderen C-Quelle als Trehalose im Medium im Gegensatz zum Wildtyp weniger oder bei weiterer Selektionierung keine Trehalose mehr anreichern. Auch wenn die nach dem ersten Selektionierungsschritt isolierten Mutanten keine Trehalose mehr ausscheiden, gelingt es trotzdem, in einem weiteren Selektionsverfahren die Ausbeute im Vergleich zum Ausgangsstamm zu erhöhen.

Es handelt sich dabei um ein alternierendes Überimpfen der Kulturen in trehalose- bzw. saccharosehaltige (in den bekannten, vergleichbaren konzentrationen) Medien, bis die Wachstumsgeschwindigkeit in Trehalose der in Saccharose enthaltenden Medien entspricht.

Die Induzierung erfolgt in der Weise, daß der Ausgangsstamm gebräuchlichen chemischen oder physikalischen Mutagenen ausgesetzt wird, z. B. MNNG: N-Methyl-N-Nitro-N-Nitrosoguanidin oder UV-Strahlung. Die Selektion der geeigneten coryneformen Bakterien, die bevorzugt den Gattungen Corynebacterium, insbesondere Corynebacterium glutamicum, oder Brevibacterium, insbesondere Brevibacterium flavum oder lactofermentum, angehören, erfolgt nach allgemein bekannten mikrobiologischen Methoden.

### Beispiele

Die Beispiele beziehen sich auf unterschiedliche L-Lysin und Trehaloseausscheidende Mutanten von Corynebacterium glutamicum (ATCC13032), die mit Hilfe chemischer Mutagene (MNNG) gewonnen wurden.

Trehalose wird im Konzentrationsbereich 0,5 bis 100 g/l, bevorzugt 1 bis 10 g/l, eingesetzt.

### Beispiel 1

DM282-2 (leu⁻, AEC^{r}) wird über Nacht in Standard I- Bouillon (Merck Art. 7882) angezogen, gegen physiologische Kochsalzlösung (0,9 % Nacl) gewaschen, mit MNNG (N, Methyl-N -Nitro-N-Nitrosuguanidin) bei LD₃₇ mutagenisiert und in ein flüssiges Selektionsmedium inokuliert. Das Selektionsmedium ist Medium BMCG ohne Glukose, supplementiert mit 5 g/l Trehalose und 100 µg/ml L-Leucin (BMCG: Liebl et al., Appl. Microbiol. Biotechnol. (1989) 32:205 bis 210). Nach 24 h Inkubation bei 33 °C wird eine Probe genommen und ein Teil in frisches Selektionsmedium inokuliert. Ein weiterer Teil der Probe wird auf CASO-Agar (Merck Art. 5458) vereinzelt. Auf Medium BMCG wird der Phänotyp der Einzelkolonien für die genetischen Marken Leucin-Auxotropie, AEC-Resistenz und Trehalose-Verwertung geprüft. Jeweils nach 24 h wird dieses Verfahren wiederholt, bis Trehalose verwertende Mutanten nachweisbar sind. Zur Prüfung der Lysinproduktion werden Trehalose verwertende Mutanten in CASO-Bouillon (Merck Art. 5459) 16 h inkubiert (250 rpm, 33 °C). Diese Suspension wird 1:10 in 9 ml Testmedium mit 88 g/l Glucose*H₂O in 100 ml-kolben mit Schikane verdünnt und 48 h inkubiert (33 °C, 250 rpm).

Nach 48 h wird die Fermentationsbrühe abzentrifugiert und die Lysinkonzentration im Überstand mittels Aminosäureanalyse und die Trehalosekonzentration im Überstand mittels HPLC bestimmt.

**Tabelle 1**

| Stamm | L-Lysin g/l | Trehalose g/l | Wuchs auf Trehalose |
|---|---|---|---|
| DM282-2 | 34,6 | 5,5 | - |
| Klon Ia3 | 36,7 | 0 | + |
| Klon Ia6 | 37,2 | 0 | + |
| DMa45 | 36,5 | 0 | + |
| Klon IIIe2 | 35,4 | 0 | + |

### Beispiel 2

DM346-1 (leu⁻, AEC^{r}, OXA^{r}) ) wird wie in Beispiel 1 mutagenisiert und selektioniert. Nach 24 h Inkubation bei 33 °C wird eine Probe genommen und ein Teil in frisches Selektionsmedium inokuliert. Ein weiterer Teil der Probe wird auf CASO-Agar (Merck Art. 5458) vereinzelt. Auf Medium BMCG wird der Phänotyp der Einzelkolonien für die genetischen Marken Leucin-Auxotrophie, AEC-Resistenz, Oxalysinresistenz und Trehalose-Verwertung geprüft. Jeweils nach 24 h wird dieses Verfahren wiederholt, bis Trehalose verwertende Mutanten nachweisbar sind.

Die Prüfung der Lysinproduktion und der Trehaloseanreicherung erfolgt wie in Beispiel 1

**Tabelle 2**

| Stamm | L-Lysin g/l | Trehalose g/l | Wuchs auf Trehalose |
|---|---|---|---|
| DM346-1 | 36,9 | 4,9 | - |
| DM548 | 42,0 | 0 | + |
| DM559 | 37,7 | 0 | + |
| DM591 | 40,3 | 0 | + |

### Beispiel 3 :

Zur weiteren relativen Erhöhung der L-Lysin-Bildung im Vergleich zu den in Beispiel 1 oder in Beispiel 2 isolierten Mutanten wurden letztere in einem variierten Verfahren nochmals gegen Trehalose selektioniert. DM545 (Beispiel 1) wird über Nacht in Medium BMCG, das statt Glucose 1 g/l Saccharose enthält undmit 100 µg/ml L-Leu supplementiert ist, bei 33 °C angezogen. Nach 24 h wird eine Probe im Verhältnis 1 : 100 in Medium BMCG, das statt Glucose 1 g/l Trehalose enthält und mit 100 µg/l L-Leucin supplementiert ist, überführt. Parallel wird ein Medium BMCG, das keinen Zucker enthält und mit 100 µg/ml L-Leucin supplementiert ist, im Verhältnis 1 : 100 angeimpft. Beide Kulturen werden bei 33 °C unter Schütteln bebrütet, bis die optische Dichte bei 660 nm der trehalosehaltigen Kultur die der zuckerfreien Kultur übersteigt. Dann wird aus der trehalosehalitgen Kultur im Verhältnis 1 : 100 in Medium BMCG, das statt Glucose 1 g/l Saccharose enthält und mit 100 µg/ml L-Leucin supplementiert ist, überimpft. Nach 24 h wird wie oben in trehalosehaltiges bzw. zuckerfreies Medium überimpft. Das alternierende Überimpfen in trehalose-, bwz. saccharosehaltige Medien wird solange fortgesetzt, bis die Wachstumsgeschwindigkeit in Trehalose der in Saccharose enthaltenden Medien entspricht. Aus dieser Kultur werden Zellen isoliert und wie in Beispiel 1 in CASO-Bouillon inkubiert. Diese Suspension wird 1 : 10 in 9 ml Testmedium mit 80 g/l Saccharose verdünnt und wie in Beispiel 1 72 h inkubiert. Die Analyse der Fermentationsbrühe erfolgt wie in Beispiel 1.

**Tabelle 3**

| Stamm | L-Lysin g/l | Trehalose g/l |
|---|---|---|
| DM282-2 | 28,15 | 4,84 |
| DM545 | 30,09 | 0 |
| DM641 | 31,15 | 0 |
| S234 | 30,78 | 0 |
| S256 | 31,14 | 0 |
| S198 | 31,52 | 0 |

### Beispiel 4

Der in Beispiel 3 beschriebene Versuch wurde mit der Änderung wiederholt, daß die Konzentration von Trehalose bzw. von Saccharose in den Selektionsmedien jeweils 10 g/l, betrug.

**Tabelle 4**

| Stamm | L-Lysin g/l | Trehalose g/l |
|---|---|---|
| DM282-2 | 28,7 | 5,2 |
| DM545 | 30,6 | 0 |
| DM646 | 33,17 | 0 |
| DM647 | 33,06 | 0 |
| DM648 | 33,02 | 0 |
| DM649 | 32,68 | 0 |
| DM653 | 33,03 | 0 |

## Patentansprüche

1. Verfahren zur Erhöhung der Leistungsfähigkeit Aminosäuren ausscheidender Stämmen coryneformer Bakterien,
dadurch gekennzeichet, daß man bei diesen Stämmen die Fähigkeit zum Wachstum auf Trehalose induziert, und gegen ein trehalosehaltiges Medium selektioniert.

2. Verfahren gemäß Anspruch 1,
dadurch gekennzeichnet, daß man alternierend gegen ein trehalose- bzw. saccharosehaltiges Medium selektioniert.

3. Verfahren gemäß Anspruch 1 oder 2
dadurch gekennzeichnet, daß man Corynebacterium einsetzt.

4. Verfahren gemäß Anspruch 1 oder 2
dadurch gekennzeichnet, daß man Brevibacterium einsetzt.

5. Verfahren gemäß einem oder mehreren der Ansprüche 1 bis 4,
dadurch gekennzeichnet, daß man L-Lysin ausscheidende Stämme einsetzte.

6. Verfahren gemäß Anspruch 5,
dadurch gekennzeichnet, daß die zu verbessernden Stämme Trehalose ausscheiden.

7. Aminosäuren ausscheidende Stämme coryneformer Bakterien mit erhöhter Leistungsfähigkeit,
dadurch gekennzeichnet, daß in ihnen die Fähigkeit zum Wachstum auf Trehalose induziert und die Mutanten gegen Trehalose-haltige Medien selektioniert wurden.

8. Stämme coryneformer Bakterien gemäß Anspruch 7 zur fermentativen Herstellung von Aminosäuren.

9. Stämme coryneformer Bakterien gemäß Anspruch 8 zur fermentativen Herstellung von L-Lysin.

## Claims

1. A process for increasing the efficiency of amino acid-excreting strains of coryneform bacteria,
characterized in that the capacity for growth on trehalose is induced in these strains and the strains are selected against a trehalose-containing medium.

2. A process according to Claim 1, characterized in that selection is carried out alternately against a trehalose-containing and a saccharose-containing medium.

3. A process according to Claim 1 or 2, characterized in that Corynebacterium is used.

4. A process according to Claim 1 or 2, characterized in that Brevibacterium is used.

5. A process according to one or more of Claims 1 to 4, characterized in that
strains excreting L-lysine are used.

6. A process according to Claim 5, characterized in that the strains to be improved excrete trehalose.

7. Amino acid-excreting strains of coryneform bacteria with increased efficiency, characterized in that the capacity for growth on trehalose has been induced in them and the mutants have been selected against trehalose-containing media.

8. Strains of coryneform bacteria according to Claim 7 for the fermentative production of amino acids.

9. Strains of coryneform bacteria according to Claim 8 for the fermentative production of L-lysine.

## Revendications

1. Procédé pour augmenter la production des souches sécrétant des aminoacides de bactéries corynéformes,
caractérisé en ce que
l'on induit dans ces souches l'aptitude à la croissance sur tréhalose, et on sélectionne contre un milieu contenant du tréhalose.

2. Procédé conformément à la revendication 1,
caractérisé en ce que
l'on sélectionne par alternance contre un milieu contenant du tréhalose ou contenant du saccharose.

3. Procédé conformément à la revendication 1 ou 2,
caractérisé en ce que
l'on met en oeuvre Corynébacterium.

4. Procédé conformément à la revendication 1 ou 2,
caractérisé en ce que
l'on met en oeuvre Brevibacterium.

5. Procédé conformément à l'une ou à plusieurs des revendications 1 à 4,
caractérisé en ce que
l'on met en oeuvre des souches sécrétant de la L-Lysine.

6. Procédé conformément à la revendication 5, caractérisé en ce que
les souches qui doivent être améliorées sécrètent du tréhalose.

7. Souches sécrétant des aminoacides de bactéries corynéformes ayant une productivité augmentée,
caractérisées en ce qu'
on induit dans celles-ci l'aptitude à la croissance sur tréhalose et en ce que l'on sélectionne les mutants contre des milieux contenant du tréhalose.

8. Souches de bactéries corynéformes conformément à la revendication 7 pour la production par fermentation d'aminoacides.

9. Souches de bactéries corynéformes conformément à la revendication 8 pour la production par fermentation de la L-Lysine.
